# EUROPEAN PATENT SPECIFICATION

(11) **EP 2 931 180 B1**
(45) Date of publication and mention of the grant of the patent: **02.11.2016**
(21) Application number: 13818876.8
(22) Date of filing: 12.12.2013
(51) Int. Cl.: A61F 2/30, A61F 2/38, A61F 2/46, B29C 45/00, B29C 45/26

(54) **METHOD AND DEVICE FOR PRODUCING TEMPORARY KNEE JOINT SPACERS WITH STEMS OF VARIOUS SIZES**
VERFAHREN UND VORRICHTUNG ZUR HERSTELLUNG PROVISORISCHER KNIEGELENK-ABSTANDSHALTER MIT SCHÄFTEN UNTERSCHIEDLICHER GRÖSSE
PROCÉDÉ ET DISPOSITIF PERMETTANT DE PRODUIRE DES ÉCARTEURS D'ARTICULATION DE GENOU TEMPORAIRES AYANT DES TIGES DE DIMENSIONS DIFFÉRENTES

(30) Priority: 12.12.2012 US 201261736386 P
(43) Date of publication of application: 21.10.2015
(60) Divisional application: 16183586.3
(73) Proprietor: Biomet Manufacturing, LLC, Warsaw, IN 46582 (US)
(72) Inventor: SMITH, Daniel B., Warsaw, Indiana 46580 (US); KREIDER, Tayler E., Warsaw, Indiana 46582 (US)
(74) Representative: Manitz, Finsterwald & Partner GbR
(86) International application number: PCT/US2013/074757
(87) International publication number: WO 2014/093672

(56) References cited:
- WO-A1-2012/158618
- DE-A1-102011 104 808
- US-A1- 2004 036 189
- US-A1- 2010 102 484

## Description

### FIELD

This disclosure relates generally to orthopedic implants for use in orthopedic surgical procedures and, more particularly, to cement molds for use in forming temporary orthopedic implants used in orthopedic surgical procedures. The closest prior art is document US 2010/0102484 A1, which defines the preamble of claim 1.

### BACKGROUND

This section provides background information related to the present disclosure which is not necessarily prior art.

A natural joint may undergo degenerative changes due to a variety of etiologies. When these degenerative changes become so far advanced and irreversible, it may ultimately become necessary to replace the natural joint with a joint prosthesis. However, due to any number of reasons, a small portion of patients that undergo such orthopedic surgical procedures suffer from infections at the surgical site and generally around the implanted joint prosthesis. In order to eradicate or clear such an infection in a two-stage reimplantation, the implanted joint prosthesis is generally removed, the site is thoroughly debrided and washed, antibiotics are applied to the infected site until the infection is eliminated, and a new revision type joint prosthesis is then implanted during a subsequent orthopedic surgical procedure. Systemic antibiotics may also act as an adjunct to local antibiotic delivery. Another technique, more popular in Europe, is the one stage reimplantation in which the prosthesis is removed, the site is debrided and washed and a new permanent implant is cemented in place using antibiotic loaded bone cement.

The currently available techniques for delivering the antibiotic to the infected joint area include mixing appropriate bone cement, such as (PMMA) poly-methyl-methacrylate, with an antibiotic, such as gentamicin, and applying the mixture to the infected joint area. Another technique involves the use of pre-loaded antibiotic cement beads which are retained on a string or wire. The antibiotic loaded bone cement is packed into the voids created by the explanted joint prosthesis while the joint is distracted or the string of antibiotic loaded beads are dropped into the respective voids. During this period, the antibiotic leaches out from the bone cement and into the infected area, while the patient may be left substantially non-ambulatory or bed-ridden with very limited mobility. In addition, soft tissue contraction in the area about the joint may cause a more difficult revision surgery since the remaining bone portion is smaller than the explanted joint prosthesis. Moreover, the above techniques may also suffer from the disadvantage of sometimes being difficult or messy to use during the orthopedic surgical procedure. This disadvantage is primarily exhibited during the use of the antibiotic loaded bone cement in a doughy state and attempting to fill the appropriate region in the distracted joint area.

### SUMMARY

A cement mold assembly configured to form a temporary tibial knee implant for use in delivering antibiotics to an infected site according to the present invention is defined in claim 1.

A method of making a temporary tibial knee implant for use in delivering antibiotics to an infected site is defined in claim 11.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is an exploded perspective view of a cement mold assembly constructed in accordance with the present invention.
FIG. 2 is a perspective view of the cement mold assembly of FIG. 1 shown in an assembled position.
FIG. 3 is a cross-sectional view taken along lines 3-3 of FIG. 2.
FIG. 4 is a cross-sectional view taken along lines 4-4 of FIG. 2.
FIG. 5 is a cross-sectional view of the cement mold assembly shown subsequent to delivery of cement within the tibial component forming cavité.
FIG. 6 is an exploded bottom perspective view of another cement mold assembly.
FIG. 7 is a top perspective view of the cement mold assembly of FIG. 6 and further illustrating an optional reinforcement member configured to be molded as part of the resulting temporary implant.
FIG. 8 is a cross-sectional view of the cement mold assembly of FIG. 6.
FIG. 9 is another cross-sectional view of the cement mold assembly of FIG. 6.
FIG. 10 is an exploded perspective view of a cement mold assembly which is not part of the present invention.
FIG. 11 is a cross-sectional view taken along lines 11-11 of FIG. 10.
FIG. 12 is a cross-sectional view of the cement mold assembly of FIG. 11 shown subsequent to removing of a plug and introduction of flowable cement.
FIG. 13 is a perspective view of an alternative femoral component cement mold assembly.
FIG. 14 is a cross-sectional view of the femoral component cement mold assembly of FIG. 13.
FIG. 15 is a cross-sectional view of a intramedullary canal filling component cement mold assembly.
FIG 16 is a partial perspective view showing an end of a stem component molded with the mold of FIG. 15.
FIG. 17 is a perspective view of an alternative tibial component cement mold assembly.
FIG 17A is a perspective view of an implant removal tool, which is similar to the plug of FIG. 17.
FIG. 18 is a cross-sectional view of the tibial component cement mold assembly of FIG. 17.

Corresponding reference numerals indicate corresponding parts throughout the several views of the drawings.

### DETAILED DESCRIPTION

Example embodiments will now be described more fully with reference to the accompanying drawings.

The following description of the preferred embodiments concerning cement molds for temporary implants used during orthopedic surgical procedures are merely exemplary in nature and are not intended to limit the disclosure or its application or uses.

Therefore, it will be understood that the present description and the claims are applicable to any appropriate bone in the body. It will be understood that the terms "spacers" and "temporary implants" are used interchangeably throughout this disclosure to refer to the same component. A non-limiting discussion of terms and phrases intended to aid understanding of the present technology is provided at the end of this Detailed Description.

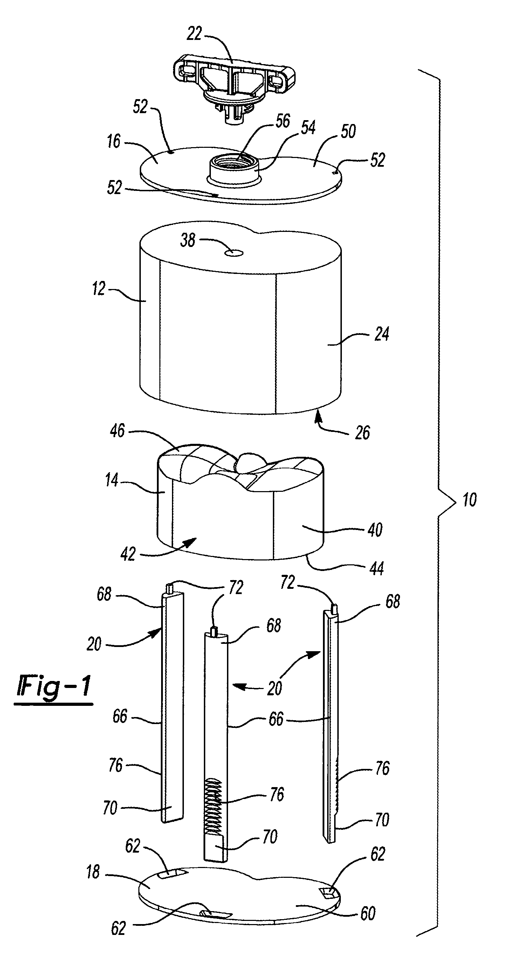

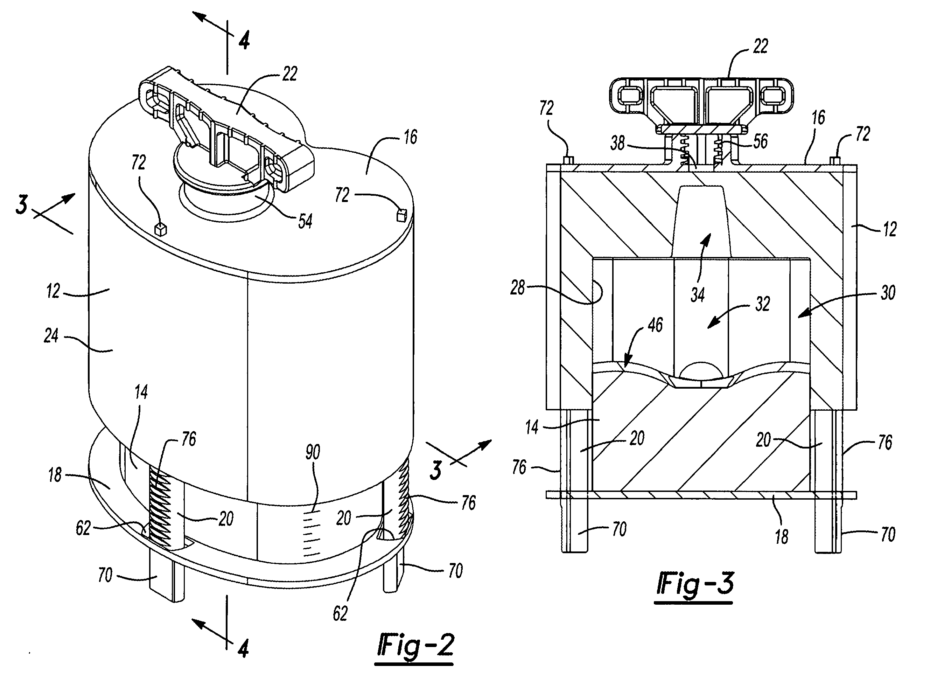

With initial reference to FIGS. 1-5, a cement mold assembly configured to form a temporary implant for use in delivering antibiotics to an infected site is shown and generally identified at reference numeral 10. The cement mold assembly 10 includes a first mold 12, a second mold 14, a first plate 16, a second plate 18, and a plurality of rods 20. The cement mold assembly 10 can further include a plug 22. As will become appreciated from the following discussion, the second mold 14 can be selectively and progressively advanced into the first mold 12 to create a unitary tibial component having a tibial tray portion and a stem portion.

The first mold 12 generally includes a first mold body 24 having an open end 26 and an inner wall 28. The first mold 12 defines a tibial component forming cavity 30 including a platform forming cavity 32 and a stem forming cavity 34. A first mold port 38 is formed through the mold body 24 and aligned with the stem forming cavity 34.

The second mold 14 includes a second mold body portion 40 having an outer wall 42, an end surface 44 and a tibial platform forming surface 46. In one example, the tibial platform forming surface 46 can include contours configured to substantially replicate a corresponding tibial bearing surface. The dimensions of the outer wall 42 of the second mold body portion 40 are slightly less than the inner wall 28 of the first mold 12 such that the second mold 14 can be selectively and progressively advanced into the tibial component forming cavity 30. In one example, the first and second molds 12 and 14 can be formed of silicone.

The first plate 16 can include a first plate body portion 50 having a plurality of apertures 52. A first plate boss 54 can extend from the first plate 16. The first plate boss 54 can include a threaded delivery port 56. The second plate 18 can include a second plate body portion 60 having a plurality of apertures 62. Each of the rods 20 can generally be formed by an elongated body portion 66 having first ends 68 and second ends 70. A projection 72 can be formed at a distal tip of each of the first ends 68.

Teeth 76 are formed along each of the elongated body portion 66. The second ends 70 can be received into the respective apertures 62 on the second plate 18. The projections 72 on the first ends 68 of the rods 20 can be received by the apertures 52 in the first plate 16. As will become appreciated herein, the teeth 76 progressively engage the second plate body portion 60 at the respective apertures 62 upon progressive advancement of the second plate 18 toward the first plate 16 (resulting in the second mold 14 being progressively received within the first mold 12).

It will be appreciated that continued advancement of the second mold 14 within the tibial component forming cavity 30 will control the resulting height or thickness of the resulting tibial platform formed by the tibial platform forming cavity 32. The thickness can be monitored such as by gradations 90 provided on the outer wall 42 of the second mold 14. The gradations 90 may be provided on other components of the cement mold assembly 10. Additionally or alternatively, a surgeon can deduce the thickness of the resulting tibial tray platform by the amount of teeth 76 that have been advanced through the respective aperture 62 on the second plate.

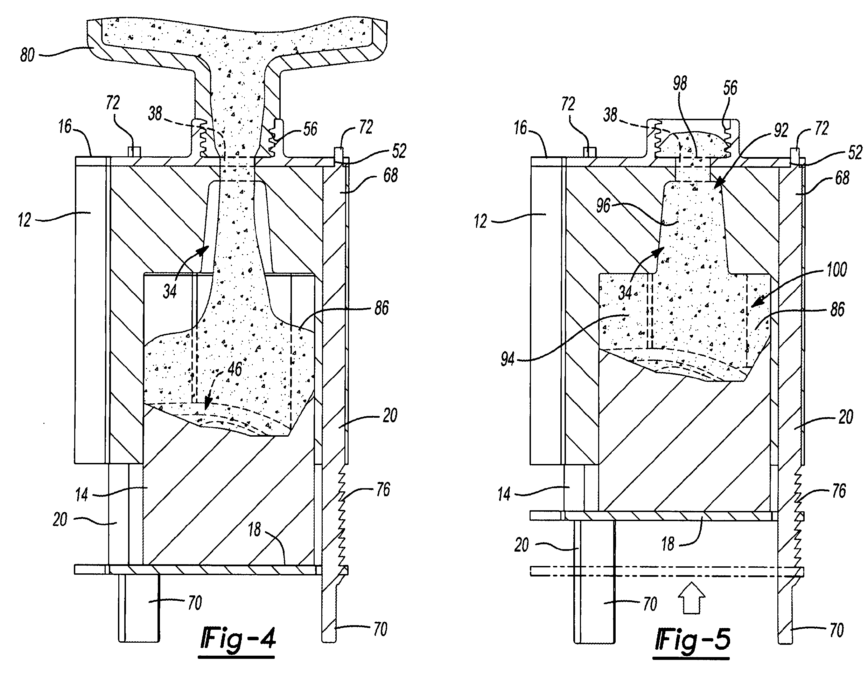

With particular reference now to FIGS. 4 and 5, a method of making a temporary implant for use in delivering antibiotics to an infected site using the mold assembly 10 will be described in greater detail. In one example, the surgeon can progressively advance the second mold 14 into the tibial component forming cavity 30 of the first mold 12 to a desired depth or cavity thickness. Then, the surgeon can couple a flowable material delivery device 80 to the threaded delivery port 56 on the first plate 16. In one example, the flowable material delivery device 80 can include a handle (not specifically shown) that can be actuated to influence emission of flowable material 86 out of the delivery device 80 to fill the tibial component forming cavity 30. Other configurations of the flowable material delivery device may be used. Furthermore, it will be appreciated that other connections may be made between the flowable material delivery device 80 and the respective port 38. For example, a luer lock coupling, a nozzle coupling or other coupling may be employed.

Once a sufficient amount of flowable material 86 has been advanced into the tibial component forming cavity 30, the flowable material delivery device 80 may be removed. Plug 22 can then be threaded onto the threaded delivery port 56 and the flowable antibiotic laden cement allowed to harden to a desired state. Thus, a partial overview of this process involves assembling the mold assembly 10, then progressively moving the first mold 12 and second mold 14 toward each other to achieve a desired or selected depth or thickness of the tibial component forming cavity 30, then filling the tibial component forming cavity 30 with flowable material and allowing it to harden sufficiently, and then removing the tibial component from the mold assembly 10.

Once the flowable material 86 has cured a sufficient amount, the mold assembly 10 can be removed from a resulting temporary implant 92. The resulting temporary implant 92 can include a tibial tray portion 94 and a stem portion 96. A portion 98 of cured flowable material 86 shown above the stem portion 96 may be discarded. The mold assembly 10 can be removed by any process such as by cutting away the first mold 12. Additionally or alternatively, a surgeon can retract the second mold 14 out of the tibial component forming cavity 30. Other methods are contemplated.

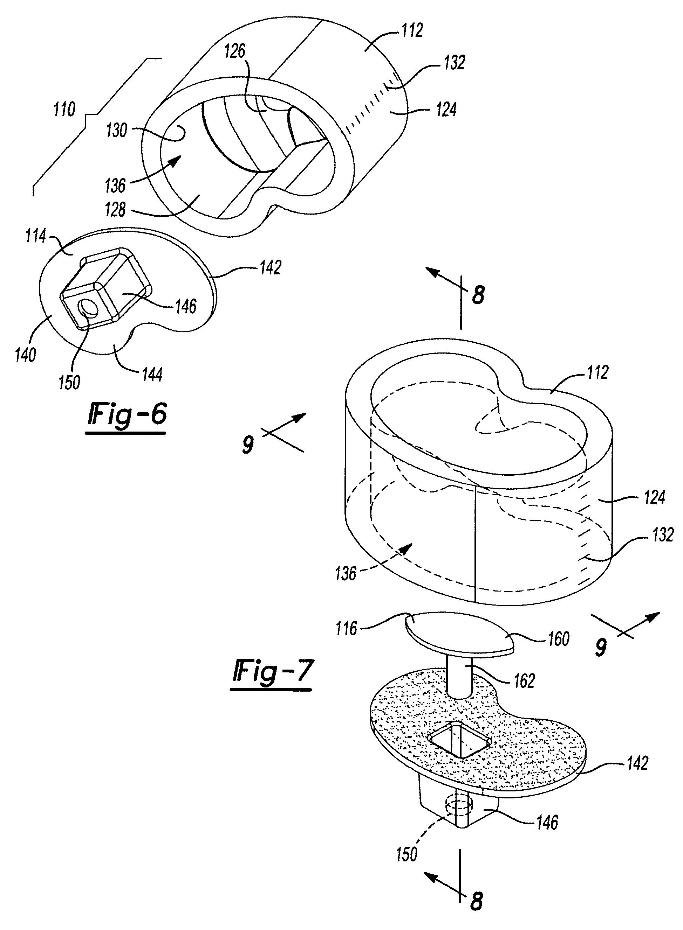

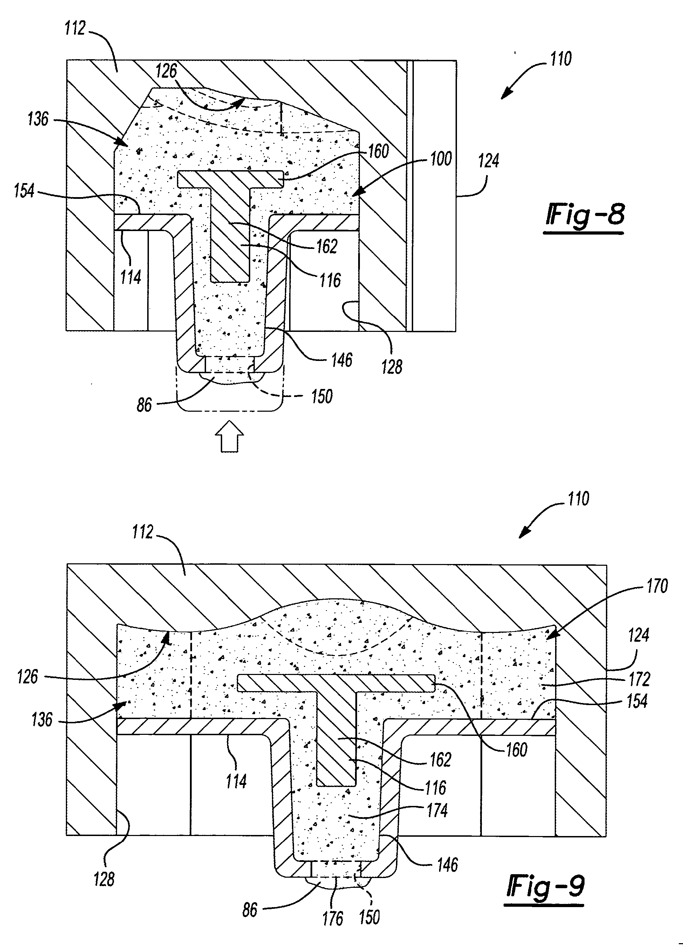

With reference now to FIGS. 6-9, a cement mold assembly configured to form a temporary implant for use in delivering antibiotics to an infected site is shown and generally identified at reference numeral 110. The cement mold assembly 110 can generally include a first mold 112 and a second mold 114. According to one example, the mold assembly 110 can further incorporate an optional reinforcing member 116 (shown in FIGS. 7-9). As will become appreciated from the following discussion, the reinforcing member 116 can be used with the mold assembly 110 in instances where a solid reinforcing structure is to be molded within the temporary implant. As will be disclosed herein, the second mold 114 can be selectively and progressively advanced into the first mold 112 to create a unitary tibial component having a tibial tray portion and a stem portion.

The first mold 112 can generally include a mold body 124 having a first closed end 126 and a second open end 128. The mold body 124 can include a sidewall 130. The sidewall 130 can include gradations 132 formed thereon. The first mold 112 can define a cavity 136. The first mold 112 can be formed of transparent or semi-transparent material to observe formation of the temporary implant. Any of the other molds disclosed herein may also be formed of transparent or semi-transparent material.

The second mold 114 can include a second mold body 140 having an outer wall 142. The second mold 114 can further include a base portion 144 and a cannulated member 146 extending therefrom. The base portion 144 can have a footprint formed by the wall 142 that is complementary to the geometry of the sidewall 130 of the first mold 112. As will become appreciated from the following discussion, the second mold 114 can be slidably and progressively received by the sidewall 130 into the cavity 136 in a direction toward the first closed end 126. The cannulated member 146 can further define an inlet port 150 defined therethrough. In the example provided, the cannulated member 146 can have a generally rectangular cross-section. The base portion 144 of the second mold 114 can include a textured end surface 154. In one example, the textured end surface 154 can facilitate removal of the resulting tibial tray from the mold assembly 110 subsequent to formation. The first mold 112 can be formed of silicone. The second mold 114 can be formed of polyethylene.

The reinforcement member 116 can include a first lateral portion 160 that can generally oppose the first closed end 126 and a second longitudinal portion 162 that is generally received by the cannulated member 146. The reinforcement member 116 can be configured to be placed at least partially within the cavity 136 prior to progressive advancement of the second mold 114 into the cavity 136. The reinforcement member 116 can be configured to be molded into the resulting unitary tibial component. It will be appreciated that the geometry of the reinforcement member 116 is merely exemplary. In this regard, the reinforcement member 116 may be shaped in any configuration. Moreover, any of the temporary implants disclosed herein may be molded with a reinforcement member therein. The reinforcement member 116 can be formed of biocompatible metal.

An exemplary method of using the mold assembly 110 according to one example will now be described. While not specifically shown, a flowable material delivery device such as described above may be coupled at the port 150 to inject flowable material into the cavity 136 between the first and second molds 112 and 114. Once a sufficient amount of flowable material 86 has been advanced into the tibial component forming cavity 136, the flowable material delivery device may be removed. Next, a surgeon can advance the second mold 114 toward the first mold 112 causing the second mold 114 to be progressively advanced into the tibial component forming cavity 136 of the first mold 112. It will be appreciated that continued advancement of the second mold 114 within the tibial component forming cavity 136 will control the resulting height or thickness of the resulting tibial platform formed by the tibial platform forming cavity 172. The thickness can be monitored such as by gradations 132 provided on the inner or outer wall 124 surface of the second mold 114. The gradations 132 may be provided on other components of the cement mold assembly 110. Additionally or alternatively, a surgeon can deduce the thickness of the resulting depth of the tibial tray cavity 172 by the depth of cavity 136 visible above the second mold 114. It will further be appreciated that during such advancement, excess flowable material 86 will be expelled through the port 150 and out of the tibial component forming cavity 30.

The second mold 114 can be advanced toward the first closed end 126 of the first mold 112 until the desired tibial tray thickness has been attained. It will be appreciated that additionally or alternatively the first mold 112 may be advanced toward the second mold 114. In some examples, a surgeon can reference the gradations 132 on the sidewall 130 of the first mold 112. The gradations 132 can be formed on the outer surface or inner surface of the sidewall 130. It will be appreciated that in some examples, excess flowable material may be expelled through the port 150.

Once the flowable material has cured a sufficient amount, the mold assembly 110 can be removed from a resulting temporary implant 170. The resulting temporary implant 170 can include a tibial tray portion 172 and a stem portion 174. A portion 176 of cured flowable material may be discarded. As identified above, the mold assembly 110 can be removed by any process.

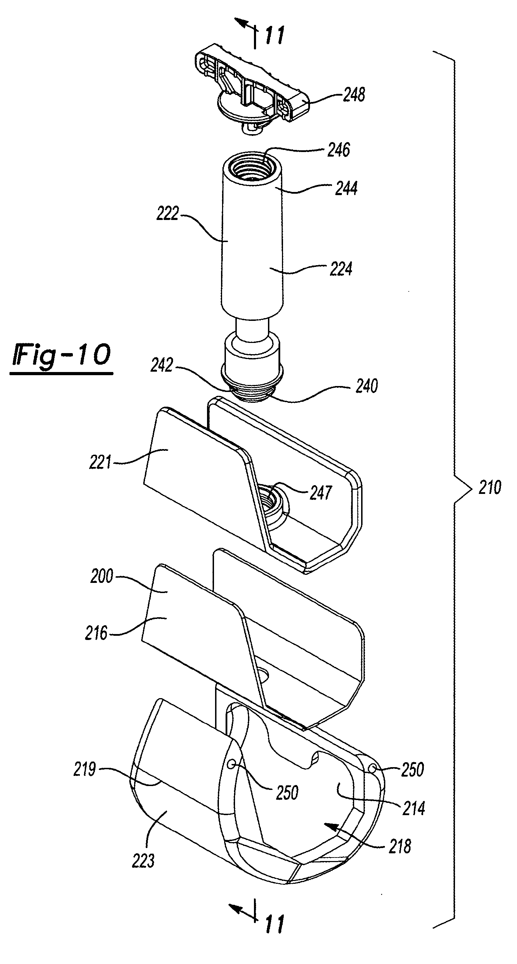

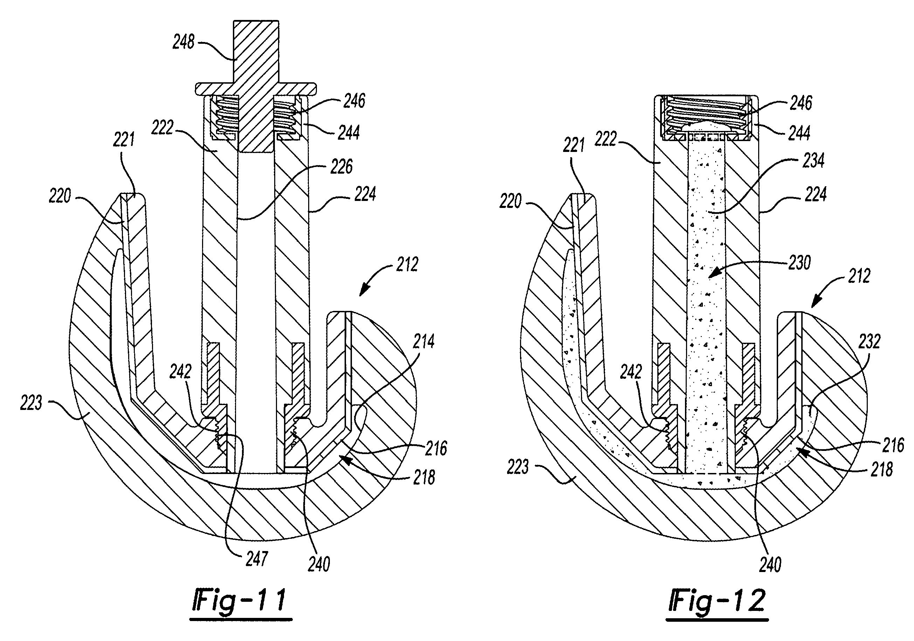

With reference now to FIGS. 10-12, a cement mold assembly configured to form a temporary implant for use in delivering antibiotics to an infected site is shown and generally identified at reference numeral 210. The cement mold assembly 210 can include a mold body 212 (FIG. 11) having an outer sidewall 214 and an inner sidewall 216 that cooperate to define a cavity 218. The inner sidewall 216 can be operable to define a shape of the temporary implant.

The mold body 212 can be collectively formed by a first mold member 219, a second mold member 220, a third mold member 221 and a fourth mold member 222. The mold body 212 can include an articulating forming portion 223 and a stem forming portion 224. The cavity 218 is collectively defined by the articulating forming portion 223 and the stem forming portion 224. Specifically, the stem forming portion 224 includes an inner surface 226 that cooperates with the inner sidewall 216 in an assembled position (FIG. 11). The articulating forming portion 223 and the stem forming portion 224 can cooperate to form a unitary femoral component 230 (FIG. 12) having an articulating portion 232 formed by the articulating forming portion 223 and a stem portion 234 formed by the stem forming portion 224.

In one example, the stem forming portion 224 can be coupled to the articulating forming portion 223. In the example shown, the stem forming portion 224 is threadably coupled to the articulating forming portion 223. The stem forming portion 224 can have a first end 240 defining threads 242 and a second end 244 defining second threads 246. The first threads 242 can be configured to threadably couple with threads 247 on the third mold member 221. The second threads 246 can be configured to threadably couple with plug 248 or a cement delivery device (not shown). Flowable material may be injected into the cavity 218 similar to that described above. In one example, the mold body 212 can define vents 250 configured to facilitate escaping air during delivery of the flowable material. It will be appreciated that vents may be additionally incorporated on any of the other molds disclosed herein. A reinforcement member, similar to the reinforcement member 116 (FIG. 7) can be optionally included as part of the cement mold assembly 210 for molding into the resulting unitary femoral component. The unitary femoral component 230 may be removed from the cement mold assembly 210.

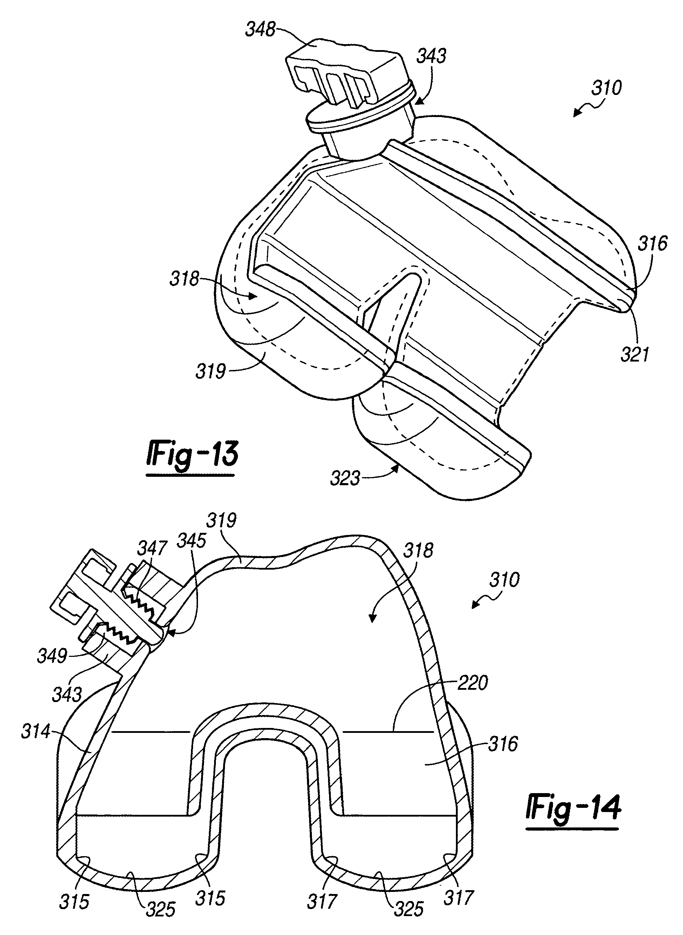

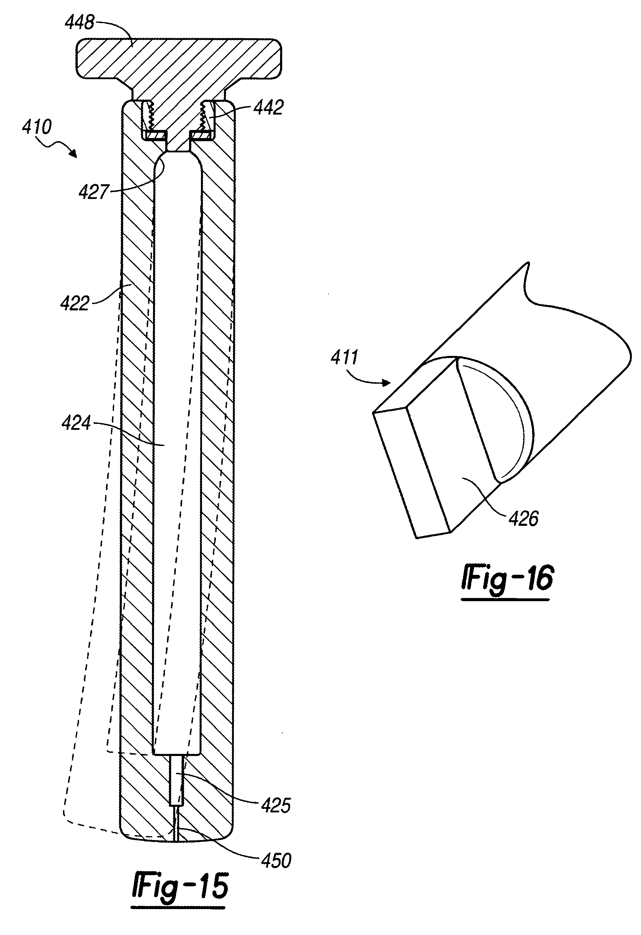

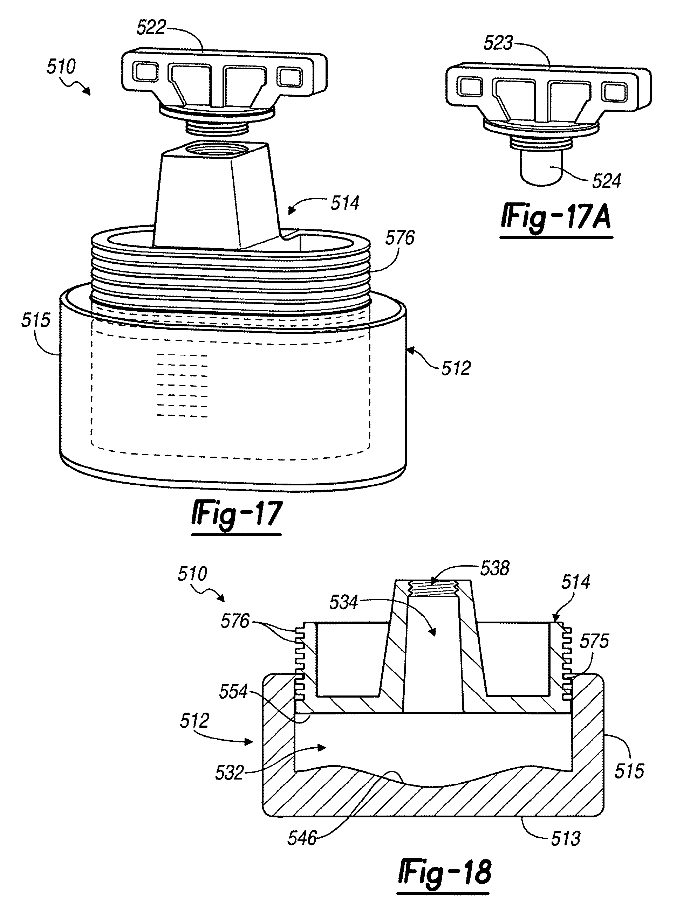

FIGS. 13-18 illustrate additional examples of various cement mold assemblies for producing antibiotic loaded cement molded components for a temporary knee joint spacer. Thus, as with the above examples, the cement mold assembly 310 of FIGS. 13 and 14 for forming the femoral knee joint spacer component, the cement mold assembly 410 of FIG 15 for forming a separate intramedullary canal filling component 411 of FIG 16, and the cement mold assembly 510 of FIGS. 17 and 18 for forming a temporary tibial component, can be provided separately or together (for example, in a kit) and can be used together in order to provide all desired antibiotic cement-molded components for a temporary knee joint spacer.

FIGS. 13 and 14 illustrate the femoral component cement mold assembly 310. This example is similar to the femoral component portion of the mold assembly 210 illustrated in FIGS. 10-12 and previously described herein. The primary differences between this femoral component mold assembly example 310 and the previously described mold assembly 210 is the cement is filled through a port or opening 345 of coupling 343 in the first mold member 319, which is the articulating forming portion 323, and this mold assembly 310 does not include any mold components for forming an integrally molded stem.

The coupling 343 is included on, and provides a port or opening 345 through, the first mold member 319, which can be made of silicone. Thus, antibiotic laden bone cement can be provided to the cavity 318 through the opening 345. The opening 345 provided in the first mold member 319 can be provided along a side so that the opening 345 is outside or away from the primary articulating surface of the molded femoral component that engages against the molded tibial component. In such a case, for example, the opening 345 into the cavity 318 would not be provided in the outer wall 314 between corners 315 and corners 317 of outer wall 314.

As seen in this example, the opening 345 intersects the cavity 318 at an angle and on the side of wall 314 between the primary tibial spacer contacting articulating surface-forming wall 325 and the femoral bone engaging or directly opposing forming wall (inner surface of inner sidewall member 316). As such, the opening 345 can be located in a substantially lateral position of the articulating surface, or - stated another way - lateral of the primary articulating surface. The opening can additionally be located in an anterior position of the articulating surface, or - stated another way - anterior of the primary articulating surface of the molded femoral component that engages against the molded tibial component.

As with the femoral component forming mold assembly 210 example of FIGS. 10-12, the bone engaging or directly opposing surface forming walls can include an inner sidewall silicone member 316 coupled against a rigid mold member 321. The outer rigid member 321 can be formed, for example, of a polycarbonate, a polyamide, or a copolyester. The inner sidewall member 316 can be made of silicone and can include vents in the corners (like vents 250 of Fig. 10) to insure complete filling of the mold cavity 318. After the mold cavity 318 is completely filled, the plug 348 can be coupled to the coupling 343 via cooperating threads 347 to seal the fill opening 345. For example, a silicone first mold member 319 can include a threaded insert of more rigid material carrying the threads 347 to cooperate with the threads 347 of the plug 348.

FIGS. 17 and 18 illustrate a cement mold assembly for forming a temporary tibial spacer component 510. This example of a tibial spacer cement mold assembly 510 can include a first mold 512, a second mold 514 that is received in the first mold 512, and a plug 522 for sealing fill port or opening 538 in the second mold member 514. In this example, the first mold member 512 includes a tibial platform forming surface 546, which forms the upper surface of the molded tibial component that engages the articulating surface of a cooperating femoral component formed using the femoral mold assembly 310.

The first mold member 512 includes inwardly projecting engagement members 575. These engagement members 575 are configured to engage cooperating engagement members 576 of the second mold member 514 as the second mold 514 is pushed into the first mold member 512 similar to that already described above with regard to the examples of FIGS. 1-5. The engagement members 575 and cooperating engagement members 576 can have a variety of shapes. As illustrated, the engagement members 575 and cooperating engagement members 576 can each include one or more projecting members that successively engage within one or more grooves between the cooperating projecting members.

In addition, the various inter-engagement members 575 and 576 and resulting grooves therebetween can have a squared configuration as seen in FIG. 18, or can have an angled or serrated configuration (similar to the teeth 76 shown in FIGS. 4 and 5). In the latter case, for example, the engagement members 575 and 576 could be angled upward (in the orientation shown in FIGS. 17 and 18) on the inner mold member 514 and angled downward on the outer mold member 512. The engagement members 575 and 576 can extend around the entire inner periphery of the first mold member 514, or can be interrupted. For example, three, four, or more separated vertical (as illustrated) regions equally spaced around the periphery can be provided with the cooperating engagement members 575 and 576. This is similar to the three rods 66, each providing one of three regions of teeth 76 around the periphery as shown, for example, in FIG. 1.

The second mold member 514 cooperates with the first mold member 512 to form a stem-forming cavity 534 and a platform-forming cavity 532. This is similar to that illustrated and described in reference to FIGS. 1-7. Also, the internal surface 554 of the second mold member 514 can be textured similar to that illustrated and described in reference to FIG. 7. The second mold member 514 can be made of any suitable material. For example, polyethylene or polypropylene can be used to form the second mold member 514.

The cooperating engagement members 576 of the second mold member 514 can be in the form of peripheral protrusions, valleys, or both. Thus, the cooperating engagement members 576 can be configured similarly to that of the engagement members 575 described above. Thus, there can be only one or more horizontal (as illustrated) level of engagement member(s) 576, and can extend continuously around the entire outer periphery, or can be discontinuous such that they are located in three, four, or more equally spaced vertical regions.

The second mold member 514 can have a threaded opening 538 providing a fill opening or passage into the mold cavity 532 and 534. Upon filling of the mold cavity 532 and 534, the plug 522 can be threaded onto the second mold member 514 to seal the fill opening 538. It should be appreciated that the tibial component mold assembly 510 of FIGS. 17 and 18, can be employed in processes that are essentially the same as those described with regard to the example of the tibial component mold assembly 10 of FIGS. 1-5 and with regard to the tibial component mold assembly 110 of Figs. 6 and 7.

After the flowable material has hardened to the desired state, the surgeon can cut the first mold member 512, or otherwise separate the first mold member 512 from the second mold member 514. This can leave the molded tibial knee component attached to the second mold member 514. This can occur in any case, but especially when the surface 554 of the second mold member 514 is textured. Plug 522 as illustrated in FIG. 17 can be removed from the threaded opening 538 and replaced with the removal tool 523 illustrated in FIG. 17A. As the removal tool 523 is threaded into the threaded opening 538, the extending protrusion 524 pushes against the adjacent surface of the tibial knee implant to push and separate it from the second mold member 514.

It should be appreciated that the nose or extending protrusion 524 has been exaggerated in the drawings and/or the threads have been shortened to emphasize the protrusion 524. In actuality, however, the cooperating threads on threaded opening 538 and the removal tool 523 will engage each other prior to the distal end of the extending protrusion 524 contacting the molded tibial implant. Thus, as the removal tool 523 continues to be threaded into the threaded opening 538, the extending protrusion 524 engages and then pushes against the adjacent surface of the tibial knee implant to push and separate it from the second mold member 514. In one example the length of the nose or extending protrusion 524 can be about 5 mm. The length of the threads of threaded opening 538, of the removal tool 523, or both can be greater than 5 mm.

FIG. 15 illustrates a separate intramedullary canal filling component mold assembly 410 and FIG. 16 partially illustrates an intramedullary canal filling component 411 molded therefrom. In some cases, there is a desire to provide a temporary intramedullary canal implant 411 formed from antibiotic-laden bone cement into an elongated cavity previously formed into the bone to accommodate a stem extending from a tibial knee component or a femoral knee component which has been removed. This might occur, for example, if an infection extends into such an intramedullary canal. The intramedullary canal filling component mold assembly 410 can prepare a separate intramedullary canal filling component 411 for such purpose.

The mold assembly 410 can include an elongated mold member 422 that can provide an elongated, cylindrical mold cavity 424. One end of the elongated cavity 424 can provide a reduced-dimension, axially extending tab-forming cavity 425. The reduced dimension tab forming cavity 425, can be configured to form a plate member 426 on one end of the molded temporary intramedullary canal implant 411 as shown in FIG. 16. A radius can be provided at the interface of the base of the plate member 426 and the end surface of the cylindrical portion of the intramedullary canal filling component 411 to help manage stresses which might otherwise cause the plate member 426 to separate therefrom. In this way, the reduced-dimension axially extending tab 426 provides an axially extending portion that can be readily grasped by a surgeon to remove the intramedullary canal implant 411 from an elongated bone cavity during a subsequent surgery.

The opposite end of the elongated mold cavity 424 can include a radiused surface 427. The radiused surface 427 can be configured to make it easier to insert the intramedullary canal filling component 411 molded from the mold assembly 410, radiused end first, into an elongated stem cavity in a bone. The mold cavity 424 can be filled through a threaded port or opening 442, which can be sealed by a threaded plug 448 after filling. In order to facilitate complete filling of the intramedullary canal filling component forming cavity 424, a vent port 450 can be provided at the end of the intramedullary canal filling component forming mold cavity 424 that is opposite the fill opening 442.

The first mold member 422 can be made from a flexible material. In this way the first mold member 422 can be angled or bent at virtually any point along its length. In fact, it can be angled or bent at a plurality of places along the length (as indicated by the dotted lines in FIG. 15. For example, the first mold member 422 can be bent to provide a intramedullary canal filling component forming cavity 424 with one or more relatively straight sections at an angle relative to one or more angled sections in order to accommodate for any variations in a preexisting bone cavity. In such a case, the first mold member 422 can be bent into an appropriate configuration or position and held in such bent position during curing of the antibiotic laden bone cement within the mold cavity 424. Such flexibility can allow the first mold member 422 to be bent to match an x-ray, for example, by laying it directly on an x-ray during curing.

After set-up of the antibiotic-laden bone cement in the mold cavity 424, the surgeon can simply cut or sever mold member 422 to remove the temporary intramedullary canal implant from the mold cavity 424. Alternatively, the first mold member 422 can comprise two sub mold members (not shown) that can be threaded together near the threaded opening 442, for example. Such sub members can simply be unscrewed in order to facilitate removal of the molded intramedullary canal filling component 411 from the mold cavity 424.

To accommodate for preexisting elongated intramedullary canals in a bone having different lengths, the mold member 422 can potentially be cut to the desired length. The cut end can be cut or otherwise shaped to have a radiused surface at its insertion end. For example, the mold 422 and cement can be cut by a surgeon before the cement has fully cured or hardened to facilitate cutting and any reshaping, or the molded intramedullary canal implant can be cut after it has been removed from the mold assembly 410.

Alternatively, a manufacturer can offer or otherwise provide a plurality of first mold members 422 or separate intramedullary canal filling component assemblies 410, each having a different axial length. Thus, a surgeon can simply choose the mold member 422 or mold assembly 422 providing the most appropriate mold cavity 424 axial length.

After removal of a molded intramedullary canal filling component 411 from the mold assembly 410, the temporary separate molded intramedullary canal filling component 411 can be inserted into the intramedullary canal. The radiused end can be inserted first. This makes the reduced dimension grasping tab 426 available for use in facilitating insertion of the separate temporary intramedullary canal filling component into the preexisting intramedullary canal bone cavity. At a point in time when it is desirable to remove the temporary intramedullary canal filling component 411, a surgeon can use the reduced-dimension grasping tab 426 to facilitate removal of the temporary intramedullary canal implant 411. For example, a surgeon can grasp the tab 426 with a surgical instrument such as pliers to pull the temporary intramedullary canal implant 411 from the intramedullary canal bone cavity.

Alternatively, the intramedullary canal implant 411 can be joined to a molded tibial component during the implantation surgery. For example, a surgeon can place antibiotic laden bone cement that is in a doughy state around plate member 426 of intramedullary canal implant 411. Upon hardening, this doughy cement can couple the intramedullary canal implant 411 to the adjacent implant, such as that formed by the mold assembly 310 or the mold assembly 510. Thus, during surgery to remove the temporary spacer implant components, the intramedullary canal implant 411 can be pulled out of the intramedullary canal along with the removal of the adjacent implant to which it has been coupled (forming a single piece integral component) during the prior implant surgery.

Providing a separate mold assembly 410 for molding a separate intramedullary canal implant 411 as described herein with reference to FIGS. 15 and 16 can permit not only bending of the first mold member 422 to match an x-ray showing the actual anatomy of the intramedullary canal of a specific individual patient, but also can permit positioning of the molded intramedullary canal implant 411 relative to the adjacent spacer implant component to match the actual anterior-posterior positioning, and the actual lateral-medial positioning of the intramedullary canal relative to the position of the adjacent implant component, which corresponds to the actual anatomy of the specific individual patient.

The foregoing description of the embodiments has been provided for purposes of illustration and description. It is not intended to be exhaustive or to limit the disclosure.

### Non-limiting Discussion of Terminology

The headings (such as "Introduction" and "Summary") and subheadings used herein are intended only for general organization of topics within the present disclosure, and are not intended to limit the disclosure of the technology or any aspect thereof. In particular, subject matter disclosed in the "Introduction" may include novel technology and may not constitute a recitation of prior art. Subject matter disclosed in the "Summary" is not an exhaustive or complete disclosure of the entire scope of the technology or any embodiments thereof. Classification or discussion of a material within a section of this specification as having a particular utility is made for convenience, and no inference should be drawn that the material must necessarily or solely function in accordance with its classification herein when it is used in any given composition.

The description and specific examples, while indicating embodiments of the technology, are intended for purposes of illustration only and are not intended to limit the scope of the technology. Moreover, recitation of multiple embodiments having stated features is not intended to exclude other embodiments having additional features, or other embodiments incorporating different combinations of the stated features. Specific examples are provided for illustrative purposes of how to make and use the compositions and methods of this technology and, unless explicitly stated otherwise, are not intended to be a representation that given embodiments of this technology have, or have not, been made or tested.

As used herein, the words "desire" or "desirable" refer to embodiments of the technology that afford certain benefits, under certain circumstances. However, other embodiments may also be desirable, under the same or other circumstances. Furthermore, the recitation of one or more desired embodiments does not imply that other embodiments are not useful, and is not intended to exclude other embodiments from the scope of the technology.

As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present technology that do not contain those elements or features.

Although the open-ended term "comprising," as a synonym of non-restrictive terms such as including, containing, or having, is used herein to describe and claim embodiments of the present technology, embodiments may alternatively be described using more limiting terms such as "consisting of" or "consisting essentially of." Thus, for any given embodiment reciting materials, components or process steps, the present technology also specifically includes embodiments consisting of, or consisting essentially of, such materials, components or processes excluding additional materials, components or processes (for consisting of) and excluding additional materials, components or processes affecting the significant properties of the embodiment (for consisting essentially of), even though such additional materials, components or processes are not explicitly recited in this application. For example, recitation of a composition or process reciting elements A, B and C specifically envisions embodiments consisting of, and consisting essentially of, A, B and C, excluding an element D that may be recited in the art, even though element D is not explicitly described as being excluded herein.

When an element or layer is referred to as being "on", "engaged to", "connected to" or "coupled to" another element or layer, it may be directly on, engaged, connected or coupled to the other element or layer, or intervening elements or layers may be present. In contrast, when an element is referred to as being "directly on", "directly engaged to", "directly connected to" or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

## Claims

1. A cement mold assembly (10) configured to form a temporary tibial knee implant for use in delivering antibiotics to an infected site, the cement mold assembly comprising:
a first mold (12) having a closed end and an inner wall extending from the closed end with the inner wall defining an open end of the first mold;
a second mold (14) receivable by the inner wall though the open end, and
the second mold having a footprint complementary to and slidable along the inner wall;
wherein the first mold cooperates with the second mold to form a cavity (30) defining the temporary tibial knee implant including a stem portion formed by a stem forming cavity (34) and a platform portion formed by a platform forming cavity (32) having a selected thickness that corresponds to any one of a plurality of platform thicknesses to be selected, **characterised in that** the cement mold assembly comprises cooperatively engaging teeth (76) operating to selectively and progressively index the first mold relative to the second mold to permit any one of the plurality of tibial platform thicknesses that is selected and includes a delivery port (56) open to the stem forming cavity.

2. The cement mold assembly (10) assembly of claim 1, wherein the first mold (12) comprises a platform forming surface of the platform forming cavity (32), and the second mold (14) comprises the stem forming cavity (34).

3. The cement mold assembly (10) of any of claims 1 and 2, wherein the first mold (12) comprises a platform forming surface of the platform forming cavity (32), and the second mold (14) comprises the stem forming cavity (34).

4. The cement mold assembly (10) of any of claims 1 through 3, wherein the closed end of the first mold (12) defines a cement fill port for the cavity (32).

5. The cement mold assembly (10) of any one of claims 1 through 4, further comprising a first plate (16) and a second plate (18) positioned outboard of the first and second molds (12, 14), respectively.

6. The cement mold assembly (10) of claim 5, further comprising at least two rods (20) connected between the first and second plates (16, 18), and wherein the at least two rods (20) and the first and second plates (16, 18) include the cooperatively engaging teeth (76).

7. The cement mold assembly (10) of any one of claims 1 to 6, wherein at least one of the first mold (12) and the second mold (14) comprises silicone and, optionally, a textured surface adjacent the stem forming cavity (34).

8. The cement mold assembly (10) of any one of claims 1 through 7, further comprising a reinforcement member (116) positioned at least partially within the cavity prior to progressively and selectively indexing the second mold (14) into the cavity, wherein the reinforcement member is positioned to be molded into the temporary tibial knee implant.

9. The cement mold assembly (10) of claim 8, wherein the reinforcement member (116) comprises a first lateral portion (160) received in the platform forming cavity (32) and a second longitudinal portion received in the stem forming cavity (34).

10. The cement mold assembly (10) of any one of claims 1 to 9, wherein at least one of the first mold (12) and the second mold (14) includes gradations (90; 132) as an aid in identifying the selected thickness.

11. A method of making a temporary tibial knee implant for use in delivering antibiotics to an infected site, the method comprising:
inserting a second mold (14) into an open end of a first mold (12) to form a cavity defining the temporary tibial knee implant including a stem portion formed by a stem forming cavity (34) and a platform portion formed by a platform forming cavity (32);
selectively and progressively indexing the first mold (12) relative to the second mold (14) until a selected one of a plurality of platform thicknesses of the platform forming cavity (32) is attained; and
inserting flowable material into the cavity through a delivery port (56) open to the stem forming cavity (32);
subsequent to curing the flowable material, removing the temporary implant from the first and second molds (12, 14);
wherein the selectively and progressively indexing of the first mold (12) relative to the second mold (14) comprises causing successive engagement of cooperatively engaging teeth (76).

12. The method of claim 11, wherein selectively and progressively indexing the first mold (12) relative to the second mold (14) urges excess cement within the cavity to be expelled out of a fill port.

13. The method of any one of claims 11 or 12, further comprising threadably coupling a flowable material delivery device (80) to a connection port.

14. The method of any one of claims 11 through 13, further comprising, prior to the progressively and selectively indexing, positioning a reinforcement member (116) at least partially within the cavity and allowing the reinforcement member (116) to be molded into the temporary tibial knee implant during curing of the flowable material.

15. The method of any one of claims 11 through 14, further comprising determining the selected one of a plurality of platform thicknesses of the tibial tray forming cavity with reference to gradations (90; 132) provided on one of the first and second molds (12, 14).

## Patentansprüche

1. Zementformbaugruppe (10), die derart konfiguriert ist, ein temporäres Tibiaknieimplantat zur Verwendung bei der Lieferung von Antibiotika an eine infizierte Stelle zu bilden, wobei die Zementformbaugruppe umfasst:
eine erste Form (12) mit einem geschlossenen Ende und einer Innenwand, die sich von dem geschlossenen Ende erstreckt, wobei die Innenwand ein offenes Ende der ersten Form definiert;
eine zweite Form (14), die von der Innenwand durch das offene Ende aufnehmbar ist, und wobei die zweite Form eine Auflagefläche aufweist, die komplementär zu der Innenwand ist und entlang dieser verschiebbar ist;
wobei die erste Form mit der zweiten Form zusammenwirkt, um einen Hohlraum (30) zu bilden, der das temporäre Tibiaknieimplantat definiert, das einen Schaftabschnitt, der durch einen Schaft bildenden Hohlraum (34) gebildet ist, und einen Plattformabschnitt aufweist, der durch einen Plattform bildenden Hohlraum (32) gebildet ist, der eine gewählte Dicke aufweist, die einer eine Mehrzahl von zu wählenden Plattformdicken entspricht, **dadurch gekennzeichnet, dass** die Zementformbaugruppe zusammenwirkend in eingriff tretende Zähne (76) umfasst, die dazu dienen, die erste Form relativ zu der zweiten Form selektiv und progressiv weiterzuschalten, um eine einer Mehrzahl von Tibiaplattformdicken, die gewählt ist, zuzulassen, und einen Lieferdurchlass (56) aufweist, der zu dem Schaft bildenden Hohlraum offen ist.

2. Zementformbaugruppe (10) nach Anspruch 1, wobei die erste Form (12) eine Plattform bildende Fläche des Plattform bildenden Hohlraums (32) umfasst und die zweite Form (14) den Schaft bildenden Hohlraum (34) umfasst.

3. Zementformbaugruppe (10) nach einem der Ansprüche 1 oder 2, wobei die erste Form (12) eine Plattform bildende Fläche des Plattform bildenden Hohlraums (32) umfasst und die zweite Form (14) den Schaft bildenden Hohlraum (34) umfasst.

4. Zementformbaugruppe (10) nach einem der Ansprüche 1 bis 3, wobei das geschlossene Ende der ersten Form (12) einen Zementfülldurchlass für den Hohlraum (32) definiert.

5. Zementformbaugruppe (10) nach einem der Ansprüche 1 bis 4, ferner mit einer ersten Platte (16) und einer zweiten Platte (18), die außerhalb der ersten bzw. zweiten Form (12, 14) positioniert sind.

6. Zementformbaugruppe (10) nach Anspruch 5, ferner mit zumindest zwei Stangen (20), die zwischen der ersten und zweiten Platte (16, 18) verbunden sind, und wobei die zumindest zwei Stangen (20) und die erste und zweite Platte (16, 18) die zusammenwirkend in Eingriff tretenden Zähne (76) aufweisen.

7. Zementformbaugruppe (10) nach einem der Ansprüche 1 bis 6, wobei zumindest eine der ersten Form (12) und der zweiten Form (14) Silikon und optional eine strukturierte Fläche benachbart des Schaft bildenden Hohlraumes (34) umfasst.

8. Zementformbaugruppe (10) nach einem der Ansprüche 1 bis 7, ferner mit einem Verstärkungselement (116), das zumindest teilweise in dem Hohlraum vor einem progressiven und selektiven Weiterschalten der zweiten Form (14) in den Hohlraum positioniert ist, wobei das Verstärkungselement zur Formung in das temporäre Tibiaknieimplantat positioniert ist.

9. Zementformbaugruppe (10) nach Anspruch 8, wobei das Verstärkungselement (116) einen ersten Seitenabschnitt (160), der an dem Plattform bildenden Hohlraum (32) aufgenommen ist, und einen zweiten Längsabschnitt umfasst, der in dem Schaft bildenden Hohlraum (34) aufgenommen ist.

10. Zementformbaugruppe (10) nach einem der Ansprüche 1 bis 9, wobei zumindest eine der ersten Form (12) und der zweiten Form (14) Abstufungen (90; 132) als eine Hilfe bei der Feststellung der gewählten Dicke aufweist.

11. Verfahren zum Herstellen eines temporären Tibiaknieimplantats zur Verwendung bei der Lieferung von Antibiotika an eine infizierte Stelle, wobei das Verfahren umfasst, dass:
eine zweite Form (14) in ein offenes Ende einer ersten Form (12) eingesetzt wird, um einen Hohlraum zu bilden, der das temporäre Tibiaknieimplantat definiert, das einen Schaftabschnitt, der durch einen Schaft bildenden Hohlraum (34) gebildet ist, und einen Plattformabschnitt aufweist, der durch einen Plattform bildenden Hohlraum (32) gebildet ist;
die erste Form (12) relativ zu der zweiten Form (14) selektiv und progressiv weitergeschaltet wird, bis eine gewählte einer Mehrzahl von Plattformdicken des Plattform bildenden Hohlraums (32) erreicht ist; und
fließbares Material in den Hohlraum durch einen Lieferdurchlass (56) eingeführt wird, der zu dem Schaft bildenden Hohlraum (32) offen ist;
anschließend an das Aushärten des fließbaren Materials das temporäre Implantat von der ersten und zweiten Form (12, 14) entfernt wird;
wobei das selektive und progressive Weiterschalten der ersten Form (12) relativ zu der zweiten Form (14) umfasst, dass ein sukzessiver Eingriff von zusammenwirkend in Eingriff tretenden Zähnen (76) bewirkt wird.

12. Verfahren nach Anspruch 11, wobei das selektive und progressive Weiterschalten der ersten Form (12) relativ zu der zweiten Form (14) überschüssigen Zement in dem Hohlraum zum Ausstoß aus einem Fülldurchlass treibt.

13. Verfahren nach einem der Ansprüche 11 oder 12, ferner umfassend, dass eine Vorrichtung (80) zur Lieferung von fließbarem Material über Gewinde mit einem Verbindungsdurchlass gekoppelt wird.

14. Verfahren nach einem der Ansprüche 11 bis 13, ferner umfassend, dass vor dem progressiven und selektiven Weiterschalten ein Verstärkungselement (116) zumindest teilweise in dem Hohlraum positioniert wird und das Verstärkungselement (116) während des Aushärtens des fließbaren Materials in das temporäre Tibiaknieimplantat geformt wird.

15. Verfahren nach einem der Ansprüche 11 bis 14, ferner umfassend, dass die gewählte einer Mehrzahl von Plattformdicken des Tibiaschalen bildenden Hohlraums in Bezug auf Abstufungen (90; 132), die an einer der ersten und zweiten Form (12, 14) vorgesehen sind, bestimmt wird.

## Revendications

1. Assemblage de moule pour ciment (10) configuré pour former un implant de genou tibial temporaire à utiliser pour la distribution d'antibiotiques à un site infecté, l'assemblage de moule pour ciment comprenant :
un premier moule (12) ayant une extrémité fermée et une paroi intérieure s'étendant depuis l'extrémité fermée, la paroi intérieure définissant une extrémité ouverte du premier moule ;
un second moule (12) susceptible d'être reçu par la paroi intérieure à travers l'extrémité ouverte, et le second moule ayant une empreinte complémentaire de et capable de coulisser le long de la paroi intérieure;
dans lequel le premier moule coopère avec le second moule pour former une cavité (30) définissant l'implant de genou tibial temporaire incluant une portion de tige formée par une cavité de formation de tige (34) et une portion de plate-forme formée par une cavité de formation de plate-forme (32) ayant une épaisseur sélectionnée qui correspond à une quelconque d'une pluralité d'épaisseurs de plate-forme à sélectionner,
**caractérisé en ce que** l'assemblage de moule pour ciment comprend des dents (76) qui s'engagent en coopération et dont la fonction est d'indexer sélectivement et progressivement le premier moule par rapport au second moule afin de permettre l'une quelconque de la pluralité d'épaisseurs de plate-forme tibiale qui est sélectionnée, et
inclut un orifice de distribution (56) ouvert vers la cavité de formation de tige.

2. Assemblage de moule pour ciment (10) selon la revendication 1, dans lequel le premier moule (12) comprend une surface de formation de plate-forme de la cavité de formation de plate-forme (32), et le second moule (14) comprend la cavité de formation de tige (34).

3. Assemblage de moule pour ciment (10) selon l'une quelconque des revendications 1 et 2, dans lequel le premier moule (12) comprend une surface de formation de plate-forme de la cavité de formation de plate-forme (32), et le second moule (14) comprend la cavité de formation de tige (34).

4. Assemblage de moule pour ciment (10) selon l'une quelconque des revendications 1 à 3, dans lequel l'extrémité fermée du premier moule (12) définit un orifice de remplissage de ciment pour la cavité (32).

5. Assemblage de moule pour ciment (10) selon l'une quelconque des revendications 1 à 4, comprenant en outre une première plaque (16) et une seconde plaque (18) positionnées à l'extérieur du premier et du second moule (12, 14) respectivement.

6. Assemblage de moule pour ciment (10) selon la revendication 5, comprenant en outre au moins deux barres (20) connectées entre la première et la seconde plaque (16, 18), et dans lequel lesdites au moins deux barres (20) et la première et la seconde plaque (16, 18) incluent les dents qui s'engagent en coopération (76).

7. Assemblage de moule pour ciment (10) selon l'une quelconque des revendications 1 à 6, dans lequel l'un au moins du premier moule (12) et du second moule (14) comprend du silicone et, en option, une surface texturée adjacente à la cavité de formation de tige (34).

8. Assemblage de moule pour ciment (10) selon l'une quelconque des revendications 1 à 7, comprenant en outre un élément de renforcement (116) positionné au moins partiellement à l'intérieur de la cavité avant d'indexer progressivement et sélectivement le second moule (14) dans la cavité, dans lequel l'élément de renforcement est positionné pour être moulé dans l'implant de genou tibial temporaire.

9. Assemblage de moule pour ciment (10) selon la revendication 8, dans lequel l'élément de renforcement (116) comprend une première portion latérale (160) reçue dans la cavité de formation de plate-forme (32) et une seconde portion longitudinale reçue dans la cavité de formation de tige (34).

10. Assemblage de moule pour ciment (10) selon l'une quelconque des revendications 1 à 9, dans lequel l'un au moins du premier moule (12) et du second moule (14) inclut des graduations (90 ; 132) à titre d'aide pour identifier l'épaisseur sélectionnée.

11. Procédé pour réaliser un implant de genou tibial temporaire à utiliser pour distribuer les antibiotiques à un site infecté, le procédé comprenant les étapes consistant à :
insérer un second moule (14) dans une extrémité ouverte d'un premier moule (12) pour former une cavité définissant l'implant de genou tibial temporaire incluant une portion de tige formée par une cavité de formation de tige (34) et une portion de plate-forme formée par une cavité de formation de plate-forme (32) ;
indexer sélectivement et progressivement le premier moule (12) par rapport au second moule (14) jusqu'à atteindre une épaisseur sélectionnée parmi une pluralité d'épaisseurs de plate-forme de la cavité de formation de plate-forme (32) ; et
insérer un matériau capable de s'écouler dans la cavité via un orifice de distribution (56) ouvert vers la cavité de formation de tige (32) ;
à la suite de la prise du matériau capable de s'écouler, enlever l'implant temporaire hors du premier et du second moule (12, 14) ;
dans lequel l'indexation sélective et progressive du premier moule (12) par rapport au second moule (14) comprend de provoquer l'engagement successif de dents qui s'engagent en coopération (76).

12. Procédé selon la revendication 11, dans lequel l'indexation sélective et progressive du premier moule (12) par rapport au second moule (14) force le ciment en excès à l'intérieur de la cavité pour le chasser hors d'un orifice de remplissage.

13. Procédé selon l'une quelconque des revendications 11 ou 12, comprenant en outre l'opération consistant à coupler par vissage un dispositif de distribution de matériau capable de s'écouler (80) à un orifice de connexion.

14. Procédé selon l'une quelconque des revendications 11 à 13, comprenant en outre, avant l'indexation progressive et sélective, le positionnement d'un élément de renforcement (116) au moins partiellement à l'intérieur de la cavité, et de permettre à l'élément de renforcement (116) d'être moulé dans l'implant de genou tibial temporaire pendant la prise du matériau capable de s'écouler.

15. Procédé selon l'une quelconque des revendications 11 à 14, comprenant en outre la détermination de l'épaisseur sélectionnée parmi une pluralité d'épaisseurs de plate-forme de la cavité de formation de plateau tibial par référence à des graduations (90 ; 132) prévues sur un moule parmi le premier et le second moule (12, 14).
